# EUROPEAN PATENT SPECIFICATION

(11) **EP 0 406 286 B1**
(45) Date of publication and mention of the grant of the patent: **13.05.1998**
(21) Application number: 89903962.2
(22) Date of filing: 23.03.1989
(51) Int. Cl.: C12N 15/29, C12N 1/20, C12N 5/02, C12P 21/02, C12P 19/34, C07K 14/00, G01N 33/531, G01N 33/532

(54) **DNA ENCODING A RYEGRASS POLLEN ALLERGEN**
DNA, DIE FÜR EIN ROGGEN-POLLEN-ALLERGEN KODIERT
ADN CODANT POUR UNE ALLERGENE DE POLLEN D'IVRAIE VIVACE

(30) Priority: 23.03.1988 AU PI7391/88
(43) Date of publication of application: 09.01.1991
(60) Divisional application: 97202850.0
(73) Proprietor: THE UNIVERSITY OF MELBOURNE, Parkville, Victoria 3052 (AU)
(72) Inventor: SINGH, Mohan, Bir, Templestowe, VIC 3106 (AU); HOUGH, Terryn, Mordialloc, VIC 3195 (AU); THEERAKULPISUT, Piyada, Carlton, VIC 3053 (AU); KNOX, Robert, Bruce, North Balwyn, VIC 3104 (AU)
(74) Representative: Bizley, Richard Edward
(86) International application number: AU8900123
(87) International publication number: WO8909260

(56) References cited:
- J. ALLERGY CLIN. IMMUNOL., vol. 81, no. 1, 1988, page 183, 44TH ANNUAL MEETING OF THE AMERICAN ACADEMY OF ALLERGY AND IMMUNOLOGY, Anaheim, CA, 11th - 16th March 1988; T.W. HATTON et al.: "Molecular cloning of kentucky bluegrass (KBG) pollen allergens"
- BIOCHEM. J., vol. 234, 1986, pages 305-310; G.P. COTTAM et al.: "Physicochemical and immunochemical characterization of allergenic proteins from rye-grass (Lolium perenne) pollen prepared by a rapid and efficient purification method"
- N. ENG. REG. ALLERGY PROC., vol. 8, no. 6, 1987, pages 409-415; R.B. MOSS: "IgG subclass antibody markers in grass pollen immunotherapy"
- JOURNAL OF CELLULAR BIOCHEMISTY, SUPPLEMENT 12C, 1988 UCLA SYMPOSIA ON MOLECULAR & CELLULAR BIOLOGY, ABSTRACTS OF THE 17TH ANNUAL MEETINGS, 28th February - 10th April 1988, page 138, abstract no. L023, Alan R. Liss, Inc., New York, US; J.P. MASCARENHAS: "Characterization of genes that are expressed in pollen"
- ADVANCES IN THE BIOSCIENCES, vol. 74, ALLERGY AND MOLECULAR BIOLOGY, DPC FIRST INTERNATIONAL SYMPOSIUM, Laguna Niguel, CA, 11th - 12th April 1988, pages 161- 172; R.B. KNOX et al.: "The rye-grass pollen allergen Lol p I"
- Journal of Allergy and Clinical Immunology Vol 78 No. 6 pp. 1190-1201, 1986 L.R. FRIEDHOFF et al., "A Study of the Human Immune Response to Lolium Perenne (Rye) Pollen and its Components, L01 pI and L01 p II (Rye I and Rye II)"
- Tissue Antigens Vol. 31 No. 4 pp 211-219 (1988) L.R. FRIEDHOFF et al., "Association of HLA-DR3 with Human Immune Response to L01 pI and L01 pII Alergens in Allergic Subjects".
- Int. Arch. Allergy, Appl. Immun. Volume 85 No. 1 pp 104-108 (1988) R.B. COOK et al "Induction of Allergen -Specific T-Cells by Conjugates of N-Formyl- Methionyl-Leucyl-Phenylalanine and Rye Grass Pollen Extract".
- CHEMICAL ABSTRACTS Vol. 108 issue No. 23 1988. WHEELLER A.W. et al "Retained T- Cell Reativity of Rye Grass Pollen Extract Following Cleavage with Cyanogen Bromide and Nitrothiocyanobenzoic Acid".
- Molecular Immunology, Volume 23, No. 12 1986 pp. 1281-1288, C.R. KAHN and D.G. MARSH, "Monoclonal Antibodies to the Major Lolium Perenne (Rye Grass) Pollen Allergen L01 pI (Rye I)".
- Immunology, Vol. 59 No. 2, 1986 pp. 309-315, R. BOSE et al., "Production and Characterization of Mouse Mono Clonal Antibodies to allergenic Epitopes on L01pI (Rye I)"
- Int. Arch. Allergy, Appl. Immun. Vol. 72 No. 3 pp. 243-248, 1983, I.J. SMART et al., "Development of Monoclonal Mouse Antibodies Specific for Allergenic Components in Ryegrass (Lolium Perenne) Pollen".
- Int. Arch. Allergy, Appl. Immun. Vol. 78, 1985 pp. 300-304, M.B. SINGH & R.B. KNOX, Grass Pollen Allergens: "Antigenic Relation Ships Detected Using Monoclonal Antibodies and Dot Blotting Immunoassay".
- Journal of Chromatography, Vol. 370 issue No. 1 pp. 165-172 (1986) BRIEVA A. and RUBIO N. "Rapid Purification of the Main Allergen of Lolium Perenne by High Performance Liquid Chromatography".

## Description

The present invention relates to a major allergenic protein from pollen of ryegrass, Lolium perenne L. (hereinafter referred to as "AP") and to derivatives and homologues thereof and to allergenic proteins immunologically related thereto. More particularly, the present invention is directed to recombinant AP, and its derivatives, and to an expression vector capable of directing synthesis of same. Even more particularly, the present invention is directed to cDNA encoding AP and to its promoter and to an expression vector comprising same.

Allergens constitute the most abundant proteins of grass pollen, which is the major cause of allergic disease in temperate climates (MARSH, 1975, HILL et al., 1979). The first descriptions of their allergenic proteins showed that they are immunochemically distinct, and are known as groups I, II, III and IV (JOHNSON AND MARSH 1965, 1966). Using the recently proposed International Union of Immunological Societies' (IUIS) nomenclature, these allergens are designated Lol pI, Lol pII, Lol pIII and Lol pIV. The major allergen, Lol pI is an acidic glycoprotein of molecular weight ca.32 kD and comprises four isoallergenic variants. The other minor allergens isolated from ryegrass pollen range in molecular weight from 10 to 76 kD (see review by FORD AND BALDO, 1986). The allergen Lol pI constitutes ca. 5% of the total extracted pollen proteins and is a glycoprotein (HOWLETT & CLARKE, 1981) containing a 5% carbohydrate moiety. Studies with carbohydrate splitting have demonstrated that the carbohydrate does not contribute to the allergic response. Allergenic activity is lost following proteolytic digestion (see review by BALDO, SUTTON & WRIGLEY, 1982), but is resistant to heat treatment, e.g. 100°C for 30 minutes at neutral pH (MARSH et al., 1966).

Lol pI is defined as an allergen because of its ability to bind to specific IgE in sera of ryegrass-sensitive patients, to act as an antigen in IgG responses, and to trigger T-cell responses. The allergenic properties have been assessed by direct skin testing of grass pollen-sensitive patients. The results showed that 84% had a skin sensitivity to Lol pI (FREIDHOFF et al., 1986), demonstrating the primary importance of this protein as the major allergen. Furthermore, 95% of patients demonstrated to be grass pollen-sensitive possessed specific IgE antibody that bound to Lol pI, as demonstrated by immunoblotting (FORD & BALDO, 1986).

Substantial allergenic cross-reactivity between grass pollens has been demonstrated using an IgE-binding assay, the radioallergo-sorbent test (RAST), for example, as described by MARSH (1970) and LOWENSTEIN (1978).

The immunochemical relationships of Lol pI with other grass pollen antigens have been demonstrated using both polyclonal and monoclonal antibodies (e.g. SMART & KNOX, 1979; SINGH & KNOX, 1985). Antibodies have been prepared to both purified proteins and IgE-binding components. These data demonstrate that the major allergen present in pollen of closely related grasses is immunochemically similar to Lol pI (SINGH & KNOX, 1985).

Further background information concerning grass pollen allergens can be found in the following reviews: MARSH (1975), HOWLETT & KNOX (1984), BALDO, SUTTON & WRIGLEY (1982) and FORD & BALDO (1986).

Recent advances in biochemistry and in recombinant DNA technology have made it possible to synthesize specific proteins, for example, enzymes, under controlled conditions independent of the organism from which they are normally isolated. These biochemical synthetic methods employ enzymes and subcellular components of the protein synthesizing systems of living cells, either in vitro in cell-free systems, or in vivo in microorganisms. In either case, the principal element is the provision of a deoxyribonucleic acid (DNA) of specific sequence which contains the information required to specify the desired amino acid sequence. Such a specific DNA sequence is termed a gene. The coding relationships whereby a deoxyribonucleotide sequence is used to specify the amino acid sequence of a protein is well-known and operates according to a fundamental set of principles (see for example, WATSON, 1976).

A cloned gene may be used to specify the amino acid sequence of proteins synthesized by in vitro systems. DNA-directed protein synthesizing systems are well-established in the art. Single-stranded DNA can be induced to act as messenger RNA (mRNA) in vitro, thereby resulting in high fidelity translation of the DNA sequence.

It is now possible to isolate specific genes or portions thereof from higher organisms, such as plants, and to transfer the genes or DNA fragments in a suitable vector, such as lambda-gt 11 phage, to microorganisms such as bacteria e.g. Escherichia coli. The transferred gene is replicated and propagated as the transformed microorganism replicates. Consequently, the transformed microorganism is endowed with the capacity to make the desired protein or gene which it encodes, for example, an enzyme, and then passes on this capability to its progeny. See, for example, Cohen and Boyer, U.S. Patent Nos. 4,237,224 and 4,468,464. The bacterial clones containing the recombinant phage are screened for the particular gene product (protein) by means of specific antibodies.

In accordance with the present invention, the gene encoding AP is cloned and thereby permitting the large scale production of recombinant allergen.

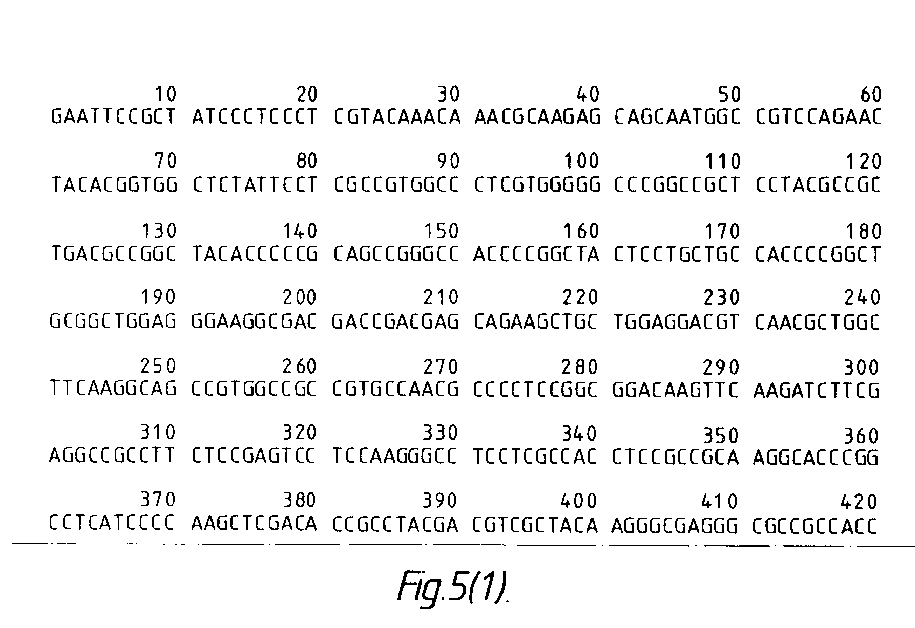

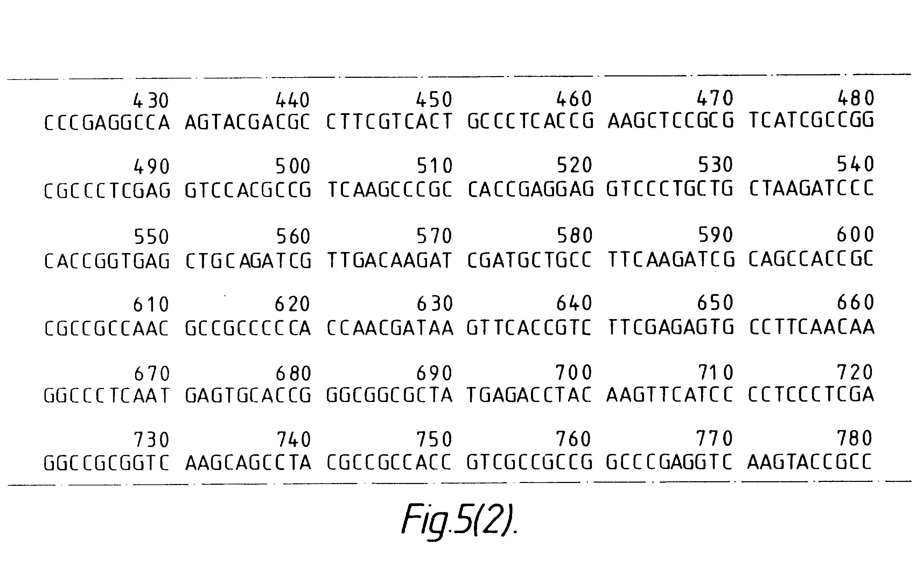

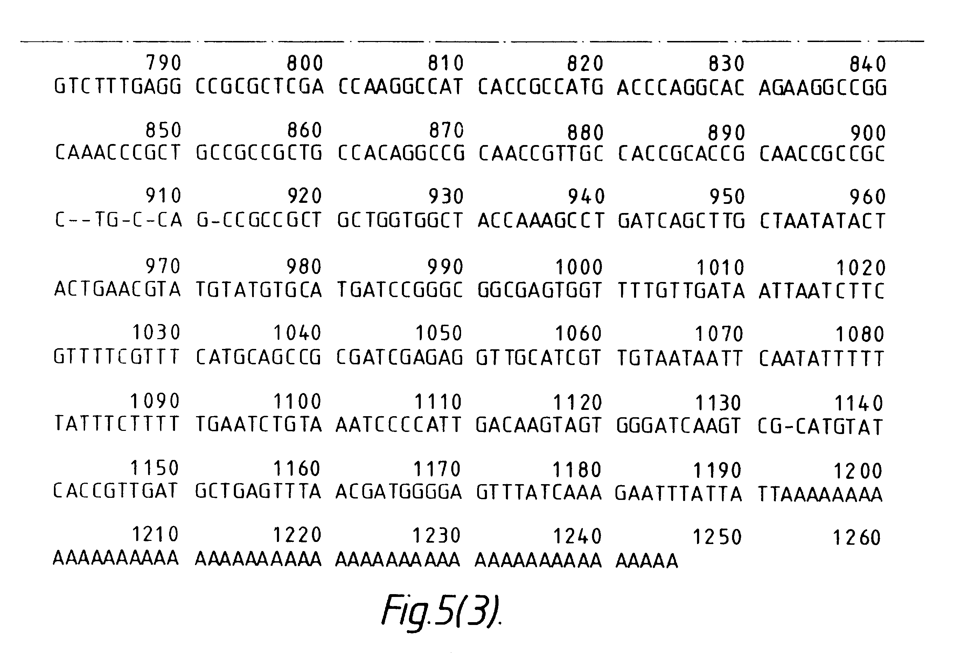

In first aspect the present invention provides a DNA sequence exhibiting at least 60% homology with the sequence depicted in Fig 5, and encoding an allergenic protein from pollen of the family Poaceae. More particularly, the grass species belongs to the family Poaceae (Gramineae), and even more particularly, to the genus Lolium. Still even more particularly, the allergenic protein is characterised as being immunologically cross-reactive with antibody to AP Lolium perenne pollen, namely:

Pooid (festucoid) grasses. GROUP 1: Triticanae: Bromus inermis, smooth brome; Agropyron repens, English couch; A. cristatum; Secale cereale rye Triticum aestivum, wheat. GROUP 2: Poanae: Dactylis glomerata, orchard grass or cocksgoot; Festuca elatior, meadow fescue; Lolium perenne, perennial ryegrass; L-multiflorum, Italian ryegrass; Poa pratensis, Kentucky bluegrass; P. compressa, flattened meadow grass; Avena sativa, oat; Holcus lanatus, velvet grass or Yorkshire fog; Anthoxanthum odoratum, sweet vernal grass; Arrhenaterum elatius, oat grass; Agrostis alba, red top; Phleum pratense, timothy; Phalaris arundinacea, reed canary grass. Panicoid grass, Paspalum notatum, Bahia grass, Andropogonoid grasses: Sorghum halepensis, Johnson grass.

The invention also provides a DNA sequence exhibiting at least 60% homology with the sequence depicted in Fig 5, and encoding an allergenic protein from a grass pollen which is allergenically cross reactive with an allergenic pollen protein from Lolium perenne as shown by IgE binding studies.

In second aspect the present invention provides an expression vector comprising a DNA sequence as defined above. More particularly, the invention provides a recombinant DNA molecule comprising a eukaryotic or prokaryotic origin of replication, a detectable marker, a DNA sequence as defined above and optionally a promoter sequence capable of directing transcription of said allergenic protein.

In third aspect the invention provides a host cell transformed to express a protein or polypeptide encoded by a DNA sequence as defined above. The host cell may be E coli.

The present invention therefore permits the production of recombinant AP immunologically reactive to antibodies to Lol pI comprising culturing a host cell containing a vector as defined above, said vector comprising a promoter capable of expression in said host cell the DNA sequence as defined above located downstream of and transcribed from said promoter, a selectable marker and a DNA vehicle containing a prokaryotic or eukaryotic origin of replication, under conditions and for a time sufficient for said recombinant DNA molecule to be stably maintained and direct the synthesis of AP.

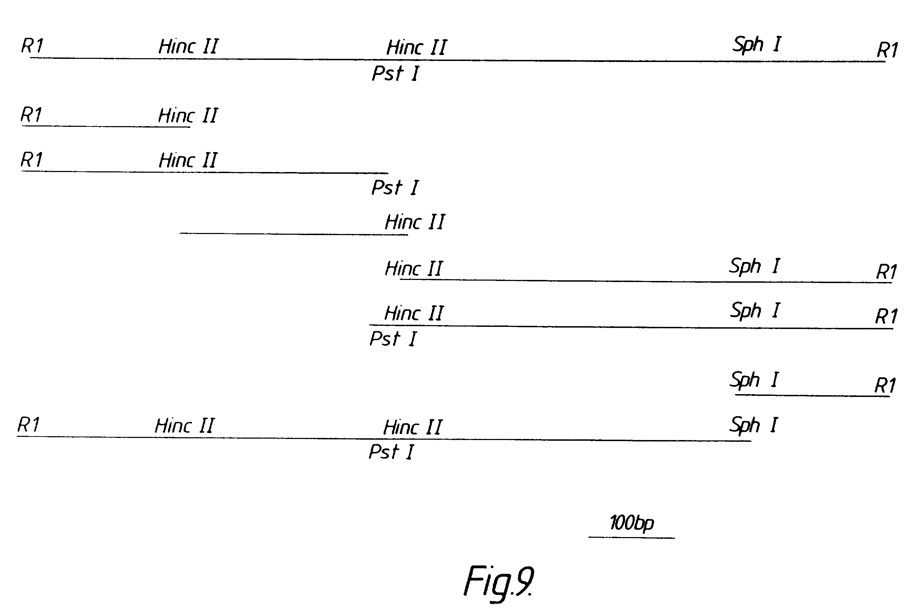

In fourth aspect the invention provides a cDNA comprising a DNA sequence as defined in any one of claims 1 to 3 and obtainable by:
extracting RNA from ryegrass pollen, and selecting Poly (A+) mRNA by affinity chromatography to serve as a template for synthesis of single-stranded cDNA;
synthesizing and isolating double stranded cDNA;
constructing a lambda-gt 11 cDNA expression library;
screening the lambda-gt 11 cDNA expression library after induction with IPTG using monoclonal antibodies characterized by *Smart et al., Int Archives of Allergy and Appl Immunol., 72, 243-248* to probe duplicate filter lifts and identifyinq and isolating positive phage plaques;
plating the plaques at low density and conducting duplicate filter lifts of said plaques;
screening said phage plaques containing clones producing protein allergen by incubating said filter lifts with allergic antisera or with said Smart et al monoclonal antibodies and identifying those clones which are positive when screened with monoclonal antibodies and positive when screened with allergic antisera;
recovering the cDNA insert from the allergic antisera and antibody positive phage and identifying by size analysis inserts of approximately 1200 base pairs;
inserting the said inserts into a plasmid vector for sequencing and expression;
sequencing said cDNA; and
comparing the restriction map of the sequenced DNA to the cDNA restriction map shown in Fig 9, and identifying those clones having substantially the same restriction map as that shown in Fig 9.

The invention also includes a method for isolating and identifying DNA encoding an allergenic protein of grass pollen comprising the step of using a cDNA as defined above as a heterologous probe to isolate the homologous cDNA clones of protein allergens from grass pollen.

In fifth aspect the invention provides the use of a DNA sequence as hereinbefore defined in the production of a protein or polypeptide encoded by the said DNA sequence.

In sixth aspect the invention provides the use of a DNA sequence as hereinbefore defined or a cDNA as hereinbefore defined as a probe for isolating and identifying nucleic acid encoding the said allergenic protein or polypeptide.

Further features of the present invention will be better understood from the following detailed description of the preferred embodiments of the invention in conjunction with the appended figures.

Standard biochemical nomenclature is used herein in which the nucleotide bases are designated as adenine (A); thymine (T); guanine (G) and cytosine (C). Other abbreviations include:-
- BSA: Bovine serum albumin
- DEPC: Diethyl pyrocabonate
- DNA: Deoxyribonucleic acid
- DTT: Dithiothreitol
- EDTA: Disodium ethylene diamine tetra-acetate
- IPTG: Isopropyl-thio-beta-D-galactopyranoside

- LB MEDIUM: Luria-Bertani medium (1% (w/v) Bactotryptone, 1% (w/v) NaCl & 0.5% (w/v) Bacto-yeast extract in water to pH 7.5).
- LIGATION BUFFER: (10 x solution)
0.66 M Tris Cl (pH 7.5) 50mM Mg Cl₂, 50mM DTT, 10mM ATP.
- LiCL: Lithium chloride.
- PEG: Polyethylene glycol.
- pfu: Plaque forming units.
- PMSF: Phenylmethylsulphonylfluoride.
- SAMPLE BUFFER: 50mM Tris-Cl, pH 6.8, 1.5% (w/v) SDS, 50mM DTT, 4M Urea, lm MPMSF.
- SDS: Sodium dodecyl sulfate.
- SM BUFFER: Phage storage buffer (0.1M NaCl, MgSO₄7H₂O, 50mM Tris HCl pH 7.5, 2% (w/v) gelatin).
- SSC: 20 x solution of 3M NaCl, 0.3M Na₃ citrate, pH 7.0.
- SSPE: (0.15M NaCl, 10mM Sodium Phosphate pH 7.7, 1mM
- EDTA: Ethylene diamine tetra-acetic acid
- TBS: Tris buffered saline (50mM Tris pH 7.5, 150 mM NaCl).
- X-gal: 5-Bromo-4-chloro 3-indolyl beta-D-galactopyranoside.

In the accompanying figures:

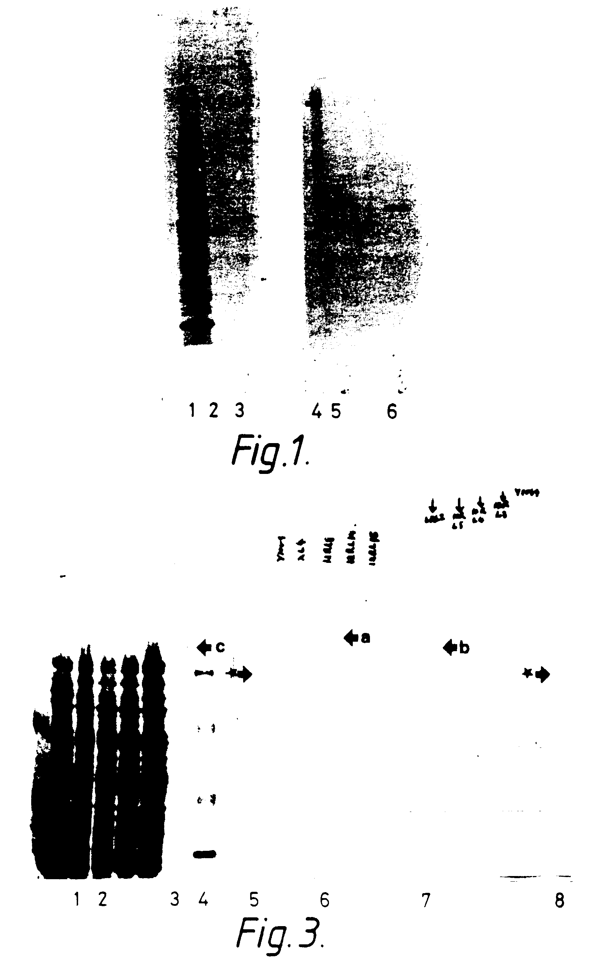

Figure 1 shows the identification of AP as the principal allergen of ryegrass pollen by SDS-PAGE and Western Blotting.

Lanes 1-3. SDS-PAGE analysis of total ryegrass pollen proteins and isolated AP allergen, stained with Coomassie blue for proteins. Lane 1, total pollen proteins; Lane 2, isoallergen of AP ; Lane 3, AP .

Lanes 4-6. Western blot of proteins shown in Lanes 1-3, showing specific binding of monoclonal antibody FMC-Al to the Lol pI allergen. This antibody was used to screen the cDNA library to select the AP allergen clones. Lane 4, molecular markers; Lane 5, isoallergen; Lane 6, AP , 32 kD.

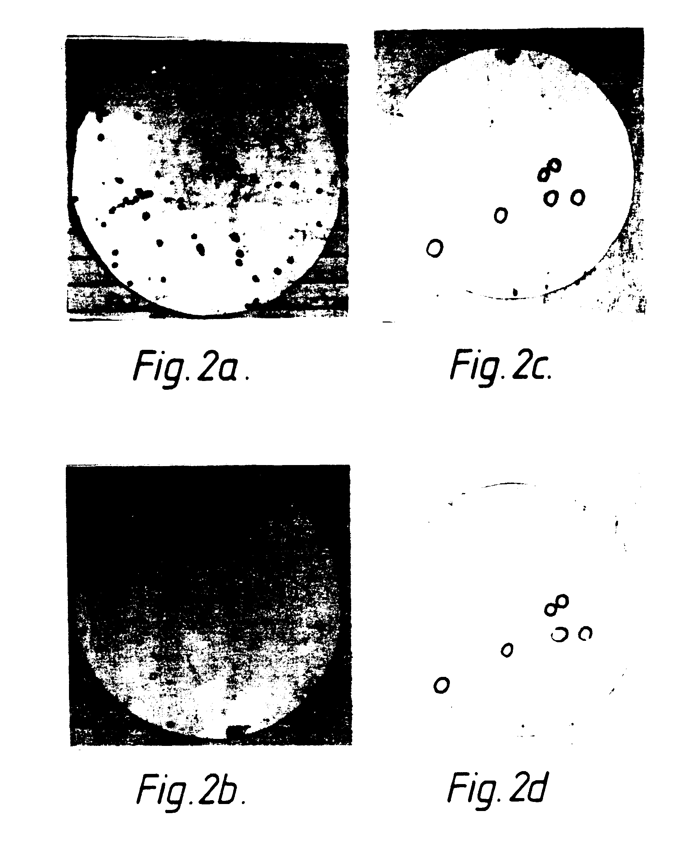

**Figure 2** shows screening of cDNA library of ryegrass pollen to select the specific clones which express AP protein in lambda-gt 11 vector. (a,b): Plaque - lifts of 10² - 10³ recombinant phages treated with specific antibody FMC-A1, with (a) cDNA clone 6; (b) cDNA clone 12; (c,d) Re-screening of cDNA clone 12 with (c) monoclonal antibody FMC-al, (d) specific IgE from ryegrass pollen-sensitive patients' sera. Recombinant phage containing the specific allergen DNA insert are detected by these methods. The antibodies detect all clones which contain the antigenic determinants of AP , while IgE binds to clones containing the allergenic determinants of Ap . All clones were monoclonal antibody FMC-Al positive, as this is the basis of the screen, while a proportion bind to IgE, as with clone 12 here.

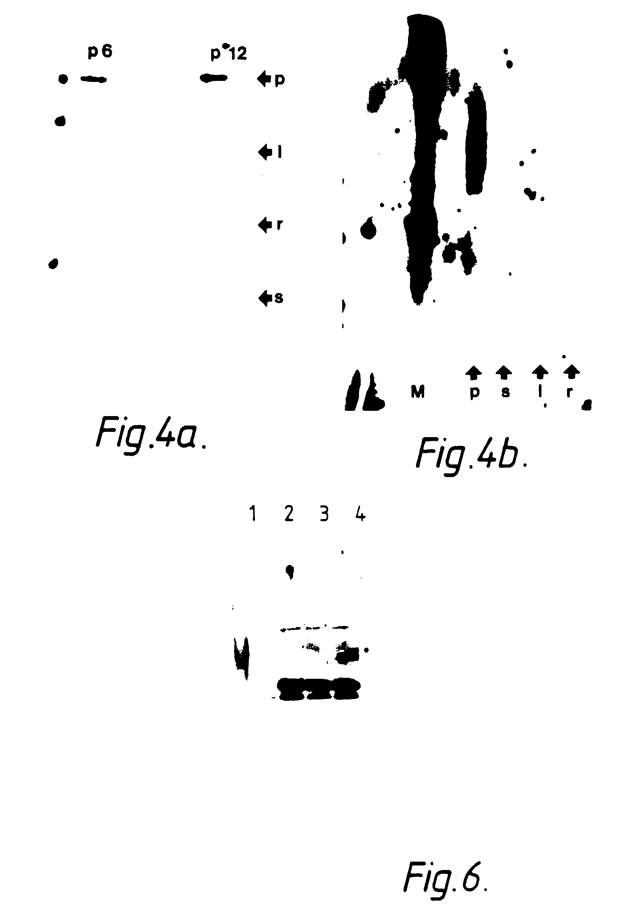

**Figure 3** shows analysis of E. coli fusion protein for identity with AP .

Lanes 1-4: SDS-PAGE stained with Coomassie blue for proteins; Lane 1, lambda-gt 11 non-recombinant lysogen extract; Lane 2, lambda-12RL8 recombinant lysogen extract; Lane 3, E. coli beta-galactosidase pure protein; Lane 4, molecular weight markers, the 96KD marker is indicated by a star.

Lanes 5-7: Transblots on nitrocellulose membrane; Lane 5 and 8, molecular weight markers; Lane 6, lambda-12RL8 recombinant lysogen extract, a indicates a fusion protein identified by binding with FMC-Al, molecular weight a>c. Lane 7, lambda-6RL2 recombinant lysogen extract, B indicates fusion protein as identified by binding of FMC-Al, molecular weight b>c. The higher molecular weights of a and b over c indicates the insertion of cDNA into the gt 11 genome at the lacZ site.

**Figure 4** shows analysis of tissue and organ specificity of AP gene in ryegrass. (a) Slot blot. 2ug each of total RNA isolated from pollen (p), leaf (1), roots (r) and hydrated seeds (s) were slot blotted onto nitrocellulose membrane. Hybridization with radioactive probes for clones 6 and 12 (p6, p12) occurs with pollen, but there is a total absence of hybridization with the other tissue RNA. (b) Northern blot. Total RNA isolated from these ryegrass tissues were separated electrophoretically in a denaturing agarose gel, and transferred to nitrocellulose membrane, and probed with p6. Hybridization occurs with the pollen sample only, other tissue RNA showing absence of hybridization. This evidence shows that AP gene is expressed only in pollen.

**Figure 5** shows a 1240 base pair DNA sequence representing the cDNA clone 12R

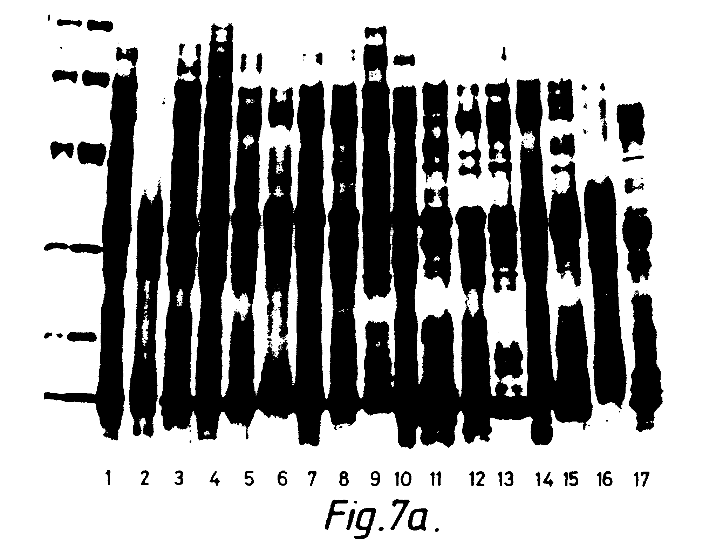

**Figure 6** shows the reaction of recombinant allergen pGEX-12R AP with IgE from pooled allergic sera. The cultures of pGEX and pGEX-12R were grown overnight and then diluted 1:10 in broth and grown for 2h at 37°C. They were induced with IPTG, and grown for lh at 37°C. The bacteria were pelletted and resuspended in PBS to 1/20 the volume of culture media. The bacteria were lysed by freeze thaw and sonication. Following that an equal volume of SDS gel sample buffer was added, and samples boiled for 3 min, before loading them onto a 10-15% gradient SDS-PAGE. The separated proteins were transferred onto nitrocellulose membrane, and these blots were processed for identification of IgE-binding proteins using pooled sera from allergic patients. ¹²⁵I-labelled anti-human IgE antibodies (Kallestad Labs USA) were used as probe. Figure 6 shows a typical autoradiograph in which lane 1 shows a vector control in which no IgE bidning is present, while lanes 2,3 and 4 show expression of recombinant AP in bacterial cultures infected with pGEX-12R.

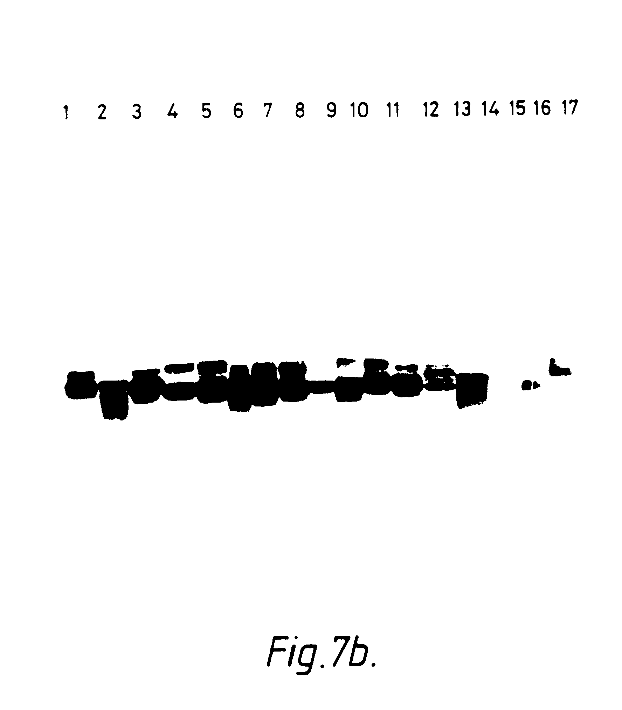

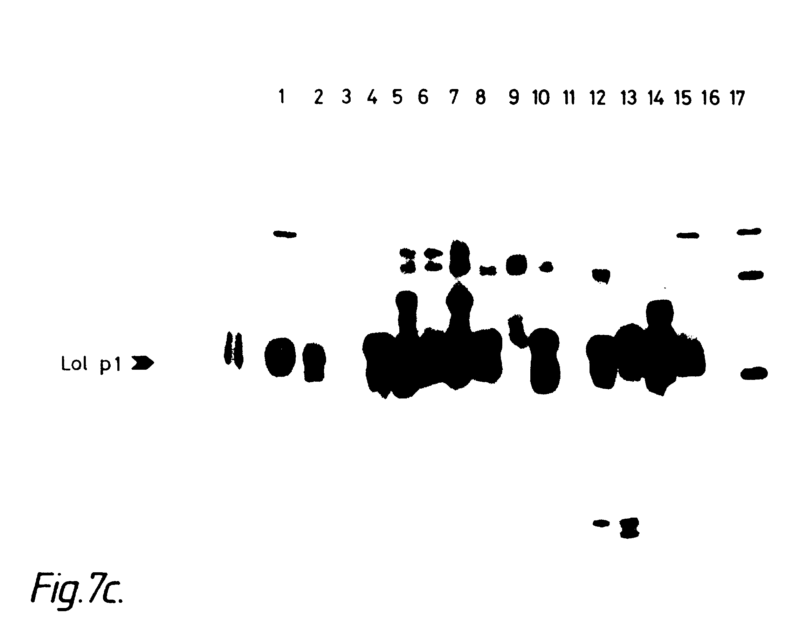

**Figure 7** A,B and C shows antigenic and allergenic similarity of proteins homologous with AP in a panel of 17 different grasses. Proteins were resolved by SDS-PAGE from mature pollen as follows: lane a: molecular weight markers; 1, Bromus inermis; 2, Agropyron cristatum, 3, Secale cereale; 4, Dactylis glomerata; 5, Festuca elatior; 6, Lolium perenne; 7, L. multiflorum; 8, Poa compressa; 9, Avena sativa; 10, Holcus lanatus; 11, Anthoxanthum odoratum; 12, Agrostis alba; 13, Phleum pratense; 14, Phalaris arundinacea; 15, Cynodon dactylon; 16, Sorghum halepensis; 17, Zea mays. 7A shows Coomassie blue stained proteins in SDS-PAGE gel. 7B shows western blot probed with monoclonal antibody FMC-Al specific for AP , showing antigenic similarity of AP and homologous allergens in related grasses, except for lane 15, Cynodon dactylon. 7C shows western blot probed with pooled allergic human sera and anti-IgE antibodies, confirming that AP and its homologous allergens in other grasses are the immunodominant allergen of grass pollen.

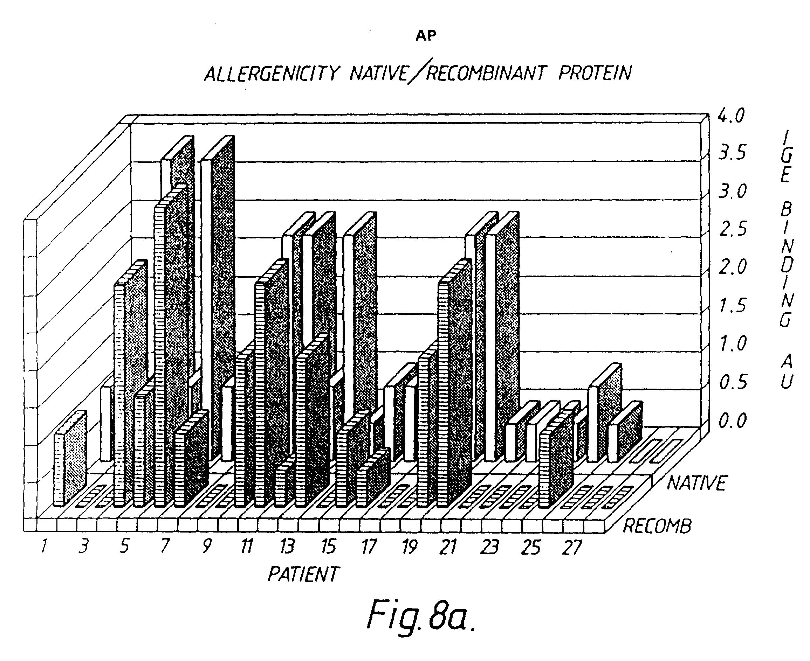

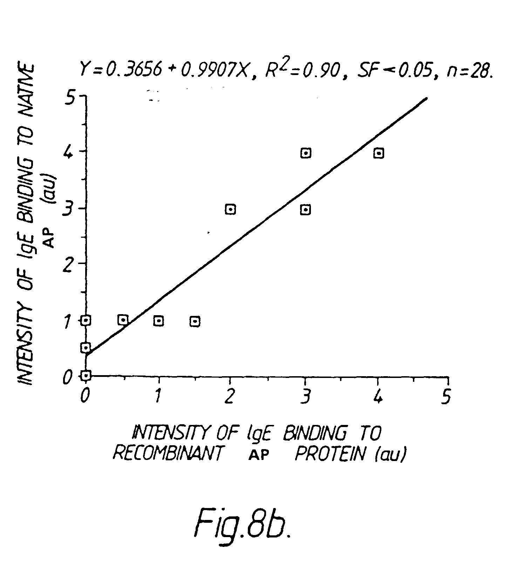

**Figure 8A** shows a comparison of allergenic activity of native and recombinant AP . Sera from 28 different patients, some of whom are allergic to grass pollen, were used to compare the IgE binding of native and recombinant AP protein. For native AP , a reference standard sample was purchased from the National Institutes of Health (NIAID), Bethesda, USA. This sample was diluted in 1% (w/v) BSA solution, and O.Sug was dot-blotted onto nitrocellulose membrane, and the blots used for IgE-binding assay. For testing IgE-binding to recombinant AP protein, the clone lambda-gt 11 -12R was expressed in host E. coli cells. The plaque lifts were used in a similar way to dot blots for testing IgE binding. Both the plaque lifts and dot blots were incubated overnight in 1:10 dilution of allergic sera, and binding of IgE visualized using rabbit anti-human IgE (Dakopatts, Copenhagen, Denmark). This incubation was followed by peroxidase-conjugated goat anti-rabbit IgG, and then the enzyme substrate to give a colour reaction. Figure 8B is a correlation of allergenic reactivity of native and recombinant AP .

**Figure 9** shows restriction map of cDNA insert to lambda-gt 11 -12R, and the strategy of nucleotide sequencing.

The original source of the genetic material is fresh ryegrass pollen from Lolium perenne L., collected from field sources near Melbourne, Australia and bulk collected pollen from a supplier (Greer Laboratories, Lenoir, NC). These sources of pollen are not intended to limit the scope of the invention since they only represent one convenient supply of the pollen. The present invention can be practised using pollen from any location. Figure 1 shows the identification of AP as the principle allergen of ryegrass pollen.

"Gene" is used herein in its broadest sense and refers to any contiguous sequence of nucleotides, the transcription of which, leads to mRNA molecule, whether or not said mRNA molecule is translatable into a polypeptide or protein. The gene encoding AP means the nucleotide sequence encoding the entire polypeptide or derivatives or homologues of said polypeptide which may contain amino acid substitutions, deletions or additions. Similarly, in relation to the carbohydrate portion of said polypeptide, derivatives include substitutions, deletions or additions to said carbohydrate moiety. The AP gene also refers to a cDNA complementary to the mRNA corresponding to the full or partial length of the AP polypeptide.

For some aspects of the present invention, it is desirable to produce a fusion protein comprising AP and an amino acid sequence from another polypeptide or protein, examples of the latter being enzymes such as beta-galactosidase, phosphatase, urease and the like. Most fusion proteins are formed by the expression of a recombinant gene in which two coding sequences have been joined together such that their reading frames are in phase. Alternatively, polypeptides can be linked in vitro by chemical means. All such fusion protein or hybrid genetic derivatives of AP or its encoding nucleotide sequence are encompassed by the present invention.

The nucleotide sequence as elucidated herein, can be used to generate any number of peptides or polypeptides by chemical synthesis, such as solid phase synthesis, by well known methods. All such chemically synthesized peptides are encompassed by the present invention. Accordingly, the present invention extends to non-native AP made by recombinant means or by chemical synthesis. Furthermore, the present invention extends to proteins, polypeptides or peptides corresponding in whole or part to the nucleotide coding sequence given in Figure 5.

It is also within the scope of the present invention to include AP immunologically cross-reactive with antibodies to AP. "Immunologically cross-reactive" is used in its broadest sense and refers generally to a protein capable of detectable binding to an antibody, the latter being specific to AP. Such an immunologically related allergen is referred to herein as a immunological relative of AP.

The cloning of the cDNA encoding AP was based on the recognition of the protein expressed by Escherichia coli transformed with lambda-gt 11 phage, using both specific monoclonal antibodies and specific serum IgE from grass pollen-sensitive patients. Two such clones are designated 6R and 12R. cDNA clones were also isolated on the basis of differential antibody binding. For example, cDNA clone 6R, was isolated on the basis that it encoded a polypeptide capable of binding to monoclonal antibodies but not IgE. Polypeptides of this type apparently lack the amino acid sequence specifying allergenicity and hence, these cDNA clones must lack the DNA sequence encoding same. Monoclonal antibodies used herein are FMC A1, A5 and A7 as described by KNOX & SINGH (1985).

For cloning the AP gene or derivatives thereof, mRNA was first isolated from the mature ryegrass pollen. This mRNA was used as a template to synthesize double stranded complementary DNAs

From this cDNA library of ryegrass pollen recombinant phage containing the AP insert were detected by screening the library with (1) specific monoclonal antibody FMC-A1; (2) specific IgE from sera of ryegrass-sensitive patients (Fig. 2).

EcoRI, linkers were then attached to both sides of selected clones of ds cDNA and then ligated into EcoRI, lambda-gt 11 vector arms (cut and dephosphorylated as purchased from Promega). The recombinant lambda-gt 11 DNA containing cDNA inserts were packaged into mature phage and the recombinant phage allowed to infect the E.coli host.

The synthesis of beta-galactosidase - recombinant gene fusion protein was induced by adding IPTG. The AP -beta-galactosidase fusion protein was then detected using monoclonal antibodies which specifically recognise the epitopes on Lol pI protein.

This fusion protein was isolated in preparative amounts from bacterial lysogens, fractionated by SDS-polyacrylamide gel electrophoresis, and the proteins transferred to nitrocellulose membranes for probing with monoclonal antibodies (Fig. 3). These antibodies recognised a protein which shows a molecular weight greater than the E. coli beta-galactosidase as would be expected of an allergen beta-galactosidase fusion protein.

The allergenic nature of the subject proteins are characterised in part, by their binding of the reaginic IgE antibodies which are present at high levels in sera of allergic patients. The IgE binding to the epitopes on allergic proteins can be tested in a chromogenic assay in which allergens immobilized on a solid support can be visualised by sequential incubation in (1) allergic patients serum; (2) enzyme-labelled anti-IgE antibodies.

Selected cDNA clones were used to probe total RNA isolated from other ryegrass plant organs to test whether AP allergen is pollen-specific or not. Slot-blotting and Northern analyses were employed (Fig. 4). No hybridization was detectable for total RNA from leaf, seed or root samples. These data indicate that AP is not expressed in these other organs of the ryegrass plant.

Selected cDNA clones were ligated into both M13 and Gemini vectors for sequencing. DNA restriction fragments to be sequenced were inserted into M13 mp14 (MESSING AND VIEIRA 1982). M13 cloning and dideoxy chain termination DNA sequencing were performed as described by Bio-rad Laboratories (1980) and MESSING (1983). A similar approach is used for the cloning of allergenic proteins from pollen of other members of the family Poaceae (Gramineae) which are immunologically cross-reactive with antibodies to Lol pI or its derivatives or homologues. The sequence of the 1240 base pair cDNA clone 12R is shown in Figure 5. It is in accordance with this invention to include or degenerate forms of said sequence and/or nucleotide sequences having substantial i.e., at least 60% homology thereto.

With this knowledge in hand, a variety of expression vectors can be constructed for the production of AP or its derivatives.

"Promoter" is used in its broadest sense and refers generally to nucleotide sequence which binds RNA polymerase and directs same to the correct transcriptional start site whereupon a gene or other nucleotide sequence thereof is transcribed. As used herein, a gene or nucleotide sequence is said to be relative to the promoter meaning that said promoter directs the transcription of the gene or nucleotide sequence. The promoter is also selected on the basis of its ability to function in a particular host. The following description relates to developing prokaryotic expression vectors capable of expressing the AP gene or a gene encoding its derivative, homologue or immunological relative, thereof. Similar principles apply for the construction of eukaryotic vectors. In this description, reference to the AP gene also includes reference to genes encoding derivative, homologues or immunological relatives of AP .

In constructing suitable prokaryotic expression vectors, transcription termination sequences are desirable to prevent potential readthrough by the RNA polymerase. To avoid any potential interference with the transcription terminators, one skilled in the art can eliminate the 3' non-coding region of the AP gene. Concurrently, one can substitute other known transcription terminators, for example, the bacteriophage lambda terminator. Thus, the present invention is in no way limited to the use of any one prokaryotic transcription terminator. Other transcription terminators include, for example, the lpp terminator and the phage SP01 terminator. All of the aforementioned terminators have been previously characterized, are well known in the art, and can be constructed either synthetically or from known plasmids.

Expression of AP activity in E. coli is in no way limited to the use or a particular promoter, since the choice of a specific promoter is not critical to the operability of this aspect of the present invention. Promoters which can be substituted for the previously exemplified λP_{L} promoter include, but are not limited to, the E.coli lactose (lac), the E.coli tryptophan (trp), the E.coli lipoprotein (lpp), and bacteriophage lambda P promoters. In addition, one or more promoters can be used in tandem, such as, for example, the trp and lac promoters, or hybrid promoters, such as the tac promoter, can be used to drive expression of the AP gene. All of the aforementioned promoters have been previously characterized, are well known in the art, and can be constructed either synthetically or from known plasmids.

The present invention is not limited to the use of any particular prokaryotic replicon. Many replicons, such as those from plasmids pBR322, pACYC184, the pUC plasmids, and the like, are known in the art and are suitable for the construction of recombinant DNA cloning and expression vectors designed to drive expression of the AP -encoding DNA compounds of the present invention. Neither is the present invention limited to the actual selectable markers present on the plasmids exemplified herein. A wide variety of selectable markers exist, both for eukaryotic and prokaryotic host cells, that are suitable for use on a recombinant DNA cloning or expression vector comprising a DNA compound (or sequence) of the present invention.

Many modifications and variations of the present illustrative DNA sequences and plasmids are possible. For example, the degeneracy of the genetic code allows for the substitution of nucleotides throughout polypeptide coding regions as well as for the substitution of the 
TAA or TGA
ATT    ACT
 translation stop signals for the 
TAG
ATC
 translational stop signal. Such sequences can be deduced from the amino acid or DNA sequence of AP and can be constructed by following conventional synthetic procedures.

The practice of this invention using prokaryotic expression vectors as well as the methods disclosed in this invention can be applied to a wide range of host organisms, especialy Gram-negative prokaryotic organisms such as Escherichia coli, E. coli K12, E. coli K12 RV308, E. coli K12 HB101, E. coli K12 C600, E. coli K12 SF8, E. coli K12 RR1, E. coli K12 RR1 M15, E. coli K12 MM294, E. coli SG936, and the like. Escherichia coli SG936 is disclosed in BUELL et al. (1985). Two of the genetic mutations introduced in this strain, the lon and htpR mutations are known to promote the expression of desired proteins (see, for example, GOFF and GOLDBERG (1985). These mutations can be transduced into other strains of E. coli by Pl transduction according to the teaching of MILLER (1972).

Alternatively, other prokaryotes can be readily employed such as Bacillus, Pseudomonas and the like. Minor modifications will need to be made to the expression vector depending on the host cell employed so that the vector replicates, the promoter functions and the selectable marker is expressed. Such modifications would be routine for one skilled in the art.

Similar considerations apply in developing eukaryotic expression vectors and many are available for use in mammalian cells, yeast and fungal cells and insect cells. A convenient reference guide to developing eukaryotic or prokaryotic expression vectors can be found in MANIATIS et al. (1982)

The AP promoter is isolatable from ryegrass genomic DNA by any number of procedures including use of promoter probes vectors, "chromosome walking" and S1 nuclease mapping and sequencing as DNA upstream of the transcription initiation site. All these techniques are well known to the skilled artisan. For example, using the cDNA clone encoding AP or its derivative as probe DNA for hybridization, a fragment of DNA adjacent to or encompassing part or all of the AP gene is cloned. The nucleotide sequence of AP as determined in accordance with the present invention, is then used, to develop nucleotide primers at the promoter-proximal end of the AP gene. "Chromosome walking", S1 endonuclease mapping, promoter probes will readily identify the promoter.

Accordingly, the present invention contemplates a recombinant DNA molecule comprising a ryegrass pollen promoter sequence, and in particular the promoter for the AP gene,
located on said molecule and further having one or more restriction endonuclease sites downstream of said promoter such that nucleotide sequence inserted into one or more of these sites is transcribeable in the correct reading frame. As used herein, the "correct reading frame" has the same meaning as "in phase". The aforementioned DNA molecule will preferably also have a selectable marker thereon, such as an antibiotic or other drug resistance gene, such as for example gene encoding resistance to ampicillin, carbenicilin, tetracycline, streptomycin and the like. The recombinant molecule will further comprise a means for stable inheritance in a prokaryotic and/or eukaryotic cell. This can be accomplished by said recombinant molecule carrying a eukaryotic and/or a prokaryotic origin of replication as hereinbefore described in relation to expression vectors.
Alternatively, the recombinant molecule will carry a means for integration into a host cell genome thereby permitting replication of said recombinant molecule in synchrony with the replication of said host cell genome. Examples of preferred prokaryotic hosts include E. coli, Bacillus and Pseudomonas amongst others. Preferred eukaryotic hosts include cells from yeast and fungi, insects, mammals and plants.

The monoclonal antibodies used in the present work to screen the cDNA library for AP clones showed cross-reactivity with allergenic proteins from pollen of various related grass species. This shows there is a homology between allergenic proteins produced by these pollens with AP allergen supporting the applicability of the present invention to all related grasses. For example, this homology can be exploited to isolate DNA encoding other allergenic proteins without the need for protein microsequencing and oligo-nucleotide primers. The present invention also relates to antibodies to recombinant AP.
Such antibodies are contemplated to be useful in developing detection assays (immunoassays) for said AP especially during the monitoring of a therapeutic or diagnostic regimen and in the purification of AP. The antibodies may be monoclonal or polyclonal. Additionally, it is within the scope of this invention to include any second antibodies (monoclonal or polyclonal) directed to the first antibodies discussed above. The present invention further contemplates use of these first or second antibodies in detection assays and, for example, in monitoring the effect of a diagnostic or an administered pharmaceutical preparation. Furthermore, it is within the scope of the present invention to include antibodies to the glycosylated regions of AP (where present), and to any molecules complexed with said AP . Accordingly, an antibody to AP encompasses antibodies to AP , or antigenic parts thereof, and to any associated molecules (e.g., glycosylated regions, lipid regions, carrier molecules, fused proteins, and the like).

The AP, or parts thereof, considered herein are purified then utilized in antibody production. Both polyclonal and monoclonal antibodies are obtainable by immunization with AP **,** and either type is utilizable for immunoassays. The methods of obtaining both types of sera are well known in the art. Polyclonal sera are less preferred but are relatively easily prepared by injection of a suitable laboratory animal with an effective amount of the purified AP , or antigenic parts thereof, collecting serum from the animal, and isolating specific sera by any of the known immunoadsorbent techniques. Although antibodies produced by this method are utilizable in virtually any type of immunoassay, they are generally less favored because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art. (See, for example, DOUILLARD, and HOFFMAN (1981) and KOHLER and MILSTEIN (1975; 1976).

Unlike preparation of polyclonal sera, the choice of animal is dependent on the availability of appropriate immortal lines capable of fusing with lymphocytes. Mouse and rat have been the animals of choice in hybridoma technology and are preferably used. Humans can also be utilized as sources for sensitized lymphocytes if appropriate immortalized human (or nonhuman) cell lines are available. For the purpose of the present invention, the animal of choice may be injected with from about 0.1 mg to about 20 mg of the purified AP , or parts thereof. Usually the injecting material is emulsified in Freund's complete adjuvant. Boosting injections may also be required. The detection of antibody production can be carried out by testing the antisera with appropriately labelled antigen. Lymphocytes can be obtained by removing the spleen or lymph nodes of sensitized animals in a sterile fashion and carrying out fusion. Alternatively, lymphocytes can be stimulated or immunized in vitro, as described, for example, in READING (1982).

A number of cell lines suitable for fusion have been developed, and the choice of any particular line for hybridization protocols is directed by any one of a number of criteria such as speed, uniformity of growth characteristics, deficiency of its metabolism for a component of the growth medium, and potential for good fusion frequency.

Intraspecies hybrids, particularly between like strains, work better than interspecies fusions. Several cell lines are available, including mutants selected for the loss of ability to secrete myeloma immunoglobulin.

Cell fusion can be induced either by virus, such as Epstein-Barr or Sendai virus, or polyethylene glycol. Polyethylene glycol (PEG) is the most efficacious agent for the fusion of mammalian somatic cells. PEG itself may be toxic for cells, and various concentrations should be tested for effects on viability before attempting fusion. The molecular weight range of PEG may be varied from 1000 to 6000. It gives best results when diluted to from about 20% to about 70% (w/w) in saline or serum-free medium. Exposure to PEG at 37°C for about 30 seconds is preferred in the present case, utilizing murine cells. Extremes of temperature (i.e., about 45°C) are avoided, and preincubation of each component of the fusion system at 37°C prior to fusion can be useful. The ratio between lymphocytes and malignant cells is optimized to avoid cell fusion among spleen cells and a range of from about 1:1 to about 1:10 is commonly used.

The successfully fused cells can be separated from the myeloma line by any technique known by the art. The most common and preferred method is to chose a malignant line which is Hypoxanthine Guanine Phosphoribosyl Transferase(HGPRT) deficient, which will not grow in an aminopterin-containing medium used to allow only growth of hybrids and aminopterin-containing medium used to allow only growth of hybrids and which is generally composed of Hypoxanthine 1x10⁻⁴M, aminopterin 1x10⁻⁵M, and thymidine 3x10⁻⁵M, commonly known as the HAT medium. The fusion mixture can be grown in the HAT-containing culture medium immediately after the fusion 24 hours later. The feeding schedules usually entail maintenance in HAT medium for two weeks and then feeding with either regular culture medium or Hypoxanthine, thymidine-containing medium.

The growing colonies are then tested for the presence of antibodies that recognize the antigenic preparation. Detection of hybridoma antibodies can be performed using an assay where the antigen is bound to a solid support and allowed to react to hydridoma supernatants containing putative antibodies. The presence of antibodies may be detected by "sandwich" techniques using a variety of indicators. Most of the common methods are sufficiently sensitive for use in the range of antibody concentrations secreted during hybrid growth.

Cloning of hybrids can be carried out after 21-23 days of cell growth in selected medium. Cloning can be formed by cell limiting dilution in fluid phase or by directly selecting single cells growing in semi-solid agarose. For limiting dilution, cell suspensions are diluted serially to yield a statistical probability of having only one cell per well. For the agarose technique, hybrids are seeded in a semisolid upper layer, over a lower layer containing feeder cells. The colonies from the upper layer may be picked up and eventually transferred to wells.

Antibody-secreting hybrids can be grown in various tissue culture flasks, yielding supernatants with variable concentrations of antibodies. In order to obtain higher concentrations, hybrids may be transferred into animals to obtain inflammatory ascites. Antibody-containing ascites can be harvested 8-12 days after intraperitoneal injection. The ascites contain a higher concentration of antibodies but include both monoclonals and immunoglobulins from the inflammatory ascites. Antibody purification may then be achieved by, for example, affinity chromatography.

The presence of AP contemplated herein, or antibodies specific for same, in a patient's serum, plant or mammalian tissue or tissue extract, can be detected utilizing antibodies prepared as above, either monoclonal or polyclonal, in virtually any type of immunoassay. A wide range of immunoassay techniques are available as can be seen by reference to U.S. Patent No. 4,016,043, 4,424,279 and 4,018,653. This, of course, includes both single-site and two-site, or "sandwich", assays of the non-competitive types, as well as in the traditional competitive binding assays. Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized in a solid substrate and the sample to be tested brought into contact with the bound molecule. After a suitable period of incubation, for a period of time sufficient to allow formation of an antibody-antigen secondary complex, a second antibody, labelled with a reporter molecule capable of producing a detectable signal is then added and incubated, allowing time sufficient for the formation of a tertiary complex of antibody-antigen-labelled antibody (e.g. antibody Lol pI antibody). Any unreacted material is washed away, and the presence of the antigen is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody, or a reverse assay in which the labelled antibody and sample to be tested are first combined, incubated and then added simultaneously to the bound antibody. These techniques are well known to those skilled in the art, including any minor variations as will be readily apparent.

Although the following discussion is concerned with detecting AP , it is equally applicable to detecting antibodies to AP and it is intended to be sufficient description thereof. In the typical forward sandwich assay, a first antibody having specificity for AP , or antigenic parts thereof, contemplated in this invention, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated at 25°C for a period of time sufficient to allow binding of any subunit present in the antibody. The incubation period will vary but will generally be in the range of about 2-40 minutes. Following the incubation period, the antibody subunit solid phase is washed and dried and incubated with a second antibody specific for a portion of the hapten. The second antibody is linked to a reporter molecule which is used to indicate the binding of the second antibody to the hapten.

By "reporter molecule," as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes). In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. For example, p-nitrophenyl phosphate is suitable for use with alkaline phosphatase conjugates; for peroxidase conjugates, 1,2-phenylenediamine, 5-aminosalicyclic acid, or toluidine are commonly used. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the tertiary complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluoresceinand rhodamine, may be chemically coupled to antibodies without altering their binding capacity. When activated by illumination with light of a particular wavelength, the fluorochrome-labelled antibody adsorbs the light energy, inducing a state of excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength, the fluorescence observed indicates the presence of the hapten of interest. Immunofluorescence and EIA techniques are both very well established in the art and are particularly preferred for the present method. However, other reporter molecules, such as radioisotope, chemiluminescent or bioluminescent molecules, may also be employed. It will be readily apparent to the skilled technician how to vary the procedure to suit the required purpose. It will also be apparent that the foregoing can be used to detect directly or indirectly (i.e., via antibodies) the Lol pI of this invention.

Because of the presence of allergens in the environment, hayfever and seasonal asthma continue to have significant morbidity and socio-economic impact on Western communities, despite advances made in their pharmacology and immunology. While the available spectrum of drugs, including anti-histamines and steroids have resulted in spectacular improvement in the treatment of allergic disease, yet they have unfortunate side-effects associated with longterm usage. Because of these problems, renewed interest has been shown in the immunotherapy of allergic disease. Immunotherapy involves the injection of potent allergen extracts to desensitize patients against allergic reactions (BOUSQUET, & MICHEL, 1989.) Unfortunately, the pollen preparations used as allergens are polyvalent and of poor quality. Consequently, concentrations used are frequently high in order to induce IgG responses, but may be lethal through triggering of systemic reactions, including anaphylaxis. The cloned gene product or synthetic peptides based on the sequence of allergens provides a safer medium for therapy since it can be quality controlled, characterized and standardized.

The precise mechanism for symptomatic relief remains hypothetical. It is established that desensitization therapy induces the formation of allergen-specific non-mast cell-binding IgG which blocks the combination of mast cell-bound IgE and allergen. This prevents mediator release, and triggering of the allergic response. Recent studies of ragweed pollen sensitivity showed that there is a correlation between allergen-specific IgG levels and relief from allergic symptoms (Lichtenstein et al., 1983). Application of reagents which can trigger allergen-specific IgG production during immunotherapy could significantly enhance the success rate of this treatment.

Currently immunotherapy is one of the most frequently administered treatments in allergology, and in USA it is considered the first choice. Advantages of this treatment for pollen rhinitis is that treatment takes up to 3 years, while pharmacotherapy must be carried out during the patient's entire life time. Patients given pollen extract for immunotherapy showed a clinical benefit that lasted for four years after the end of treatment (GRAMMER et al., 1984).

Accordingly, AP , its derivatives, homologues or immunological relatives is useful in developing a vaccine to desensitized humans to allergies due to grass pollen.

The active ingredients of a pharmaceutical composition comprising AP are contemplated to exhibit excellent therapeutic activity, for example, in the desensitization of humans allergic to grass pollen when administered in amount which depends on the particular case. For example, from about 0.5 ug to about 20 mg per kilogram of body weight per day may be administered. Dosage regima may be adjusted to provide the optimum therapeutic response. For example, several divided doses may be administered daily or the dose may be proportionally reduced as indicated by the exigencies of the therapeutic situation. The active compound may be administered in a convenient manner such as by the oral, intraveneous (where water soluble), intramuscular, subcutaneous, intranasal, intradermal or suppository routes or implanting (eg using slow release molecules). Depending on the route of administration, the active ingredients which comprise AP may be required to be coated in a material to protect said ingredients from the action of enzymes, acids and other natural conditions which may inactivate said ingredients. For example, the low lipophilicity of AP will allow it to be destroyed in the gastrointestinal tract by enzymes capable of cleaving peptide bonds and in the stomach by acid hydrolysis. In order to administer AP by other than parenteral administration, they will be coated by, or administered with, a material to prevent its inactivation. For example, AP may be administered in an adjuvant, co-administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEP) and trasylol. Liposomes include water-in-oil-in-water CGF emulsions as well as conventional liposomes.

The active compounds may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene gloycol, and the like), suitable mixtures thereof, and vegetable oils. The proper fluidity can be maintained, for example, by the use of a coating such as licithin, by the maintenance of the required particle size in the case of dispersion and by the use of surfactants. The preventions of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When AP is suitably protected as described above, the active, compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 10 ug and 2000 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavoring agent such as peppermint, oil of wintergree, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier" includes any and all solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 0.5 µg to about 2000 mg. Expressed in proportions, the active compound is generally present in from about 0.5 µg to about 2000 mg/ml of carrier. In the case of compositions containing supplementary active ingredients, the dosages are determined by reference to the usual dose and manner of administration of the said ingredients.

The present invention is further illustrated by the following non-limiting examples.

### EXAMPLE 1

### Extraction of RNA

4g of fresh ryegrass pollen (collected from field sources near Melbourne and stored under liquid N₂) was suspended in 10 ml of extraction buffer (50mM Tris buffer, pH9, 0.2M NaCl, 10mM Mg acetate), containing vanadyl ribonucleoside complexes to 10mM (BERGER AND BIRKENMEIER, 1979) and DEPC to 0.1%. The pollen sluchs was ground in a mortar and pestle under liquid N₂ for 10-20 min to provide a homogenate in which all pollen grains are broken. The slurry was transferred to Nalgene centrifuge tubes with 1% (w/v) SDS, 10mM EDTA and 0.5% (w/v) N-lauroyl sarcosine. An equal volume of warm, high grade buffered phenol (from IBI), treated with 0.1% (w/v) hydroxyquinoline (MANIATIS et al., 1982) was added, and the mixture shaken for 10 min. An equivalent volume of 24:1 parts chloroform:isoamylalcohol was added and shaking continued. Tubes were centrifuged at 15,000 rpm for 20 min at 10°C to separate the phases and remove the insoluble material and cell debris. The aqueous phase was reextracted with P:C:I four times until the phenol phase remained clear, (with phase separation at 2,500 rpm for 15 min at room temperature), and the aqueous phase was transferred to Corex centrifuge tubes. 2.5 volumes of 100% (v/v) ethanol were added and the solution mixed by pipette, and allowed to precipitate overnight at -20°C, and spun at 15,000 rpm for 20 min at 0°C. The pellet was resuspended in 10 ml of EDTA to remove the vanadyl-ribonucleaoside complexes, and LiCl added to give a final concentration of 2M. The solution was kept at 0°C overnight and centrifuged at 15,000 rpm for 30 min at 4°C. The pellet was washed with cold 2M LiCl and 5mM EDTA (pH 7.3), the liquid poured off, and the pellet resuspended in 1 ml of water. The solution is heated to 65°C, and 0.1 ml of 3M Na- acetate and 2.2 ml of ethanol were added for overnight precipitation of total RNA at -20°C. The pellet was washed gently with 70% (v/v) ethanol vacuum-dried, and resuspended in 0.5 ml water. The suspension was stored at -70°C until required for poly (A+) RNA selection.

One gram of ryegrass pollen contained 1 mg total RNA. Poly (A+) mRNA was selected by affinity chromatogrpahy on Poly (U)-Sepharose (Pharmacia ) according to standard methods. The integrity of poly (A+) mRNA was examined in terms of tis ability to act as a template for synthesis of single-stranded cDNA as well as its translational activity in the rabbit reticulocyte system.

### EXAMPLE 2

### Preparation of cDNA clones

Synthesis of first strand cDNA was from 5ug (poly A+) RNA in 50 ul reaction buffer (50mM Tris biffer, pH 8.3, containing 40mM KCl, 10mM MgCl₂, 5mM DTT, 1mM each of dATP, dGTP, dTTP, and dCTP, 50 units of human placental ribonuclease inhibitor (HPRI), 5 ug of oligodeoxythymidylic acid primer, 80 uCi of [Alpha-^{3 2} P] dCTP (3000Ci/mmol;Amersham) and 100 units of reverse transcriptase. The mixture was incubated for 60 min at 42°C. Second strand cDNA was synthesized using the mRNA/cDNA hybrids as substrate, 4 units of E. coli DNA ribonuclease H to produce nicks in the mRNA template, and 115 units of E. coli DNA polymerase 1 to catalyse the replacement of the mRNA strand by DNA. The reaction mixture was incubated sequentially at 12°C for 60 min each, and the reaction stopped by heating at 70°C for 10 min. 10 units of T4 DNA polymerase were added to remove small 3' overhangs from the first strand cDNA (GUBLER AND HOFFMAN, 1983). The reaction was stopped by adding one tenth volume of 20mM EDTA and 1% (w/v) SDS. The double strand (ds) cDNA was purified by phenol/chloroform extraction followed by precipitation with ethanol.

In order to construct a lambda-gt 11 cDNA expression library, 500 ng of double stranded cDNA was incubated with 20 units of EcoRl methylase at 37°C for 60 min.

lug of phosphorylated EcoRl linkers (5'd[pGGAATTCC]) was ligated to the double stranded cDNA in ligation buffer with 5 units of T4 DNA ligase at 15°C overnight. The EcoRl-linkered cDNA was digested with 100 units of EcoRl linkers through a Sephacryl column.

A 50ng of linkered cDNA was ligated to lug of dephosphorylated EcoRl-cut lambda-gt 11 DNA (Promega) with 2.5 units of T4 DNA ligase for 20 H at 15°C in a total vlume of 10ul. The ligated lambda-gt 11 DNA was precipitated with 30 mM Na-acetate and 2.7 volumes of ethanol at -70°C for 2h. The lambda-gt 11 DNA ligated to cDNA was packaged in vitro at 20°C for 2 h using 25 ul of the lambda packaging mixture (Promega). The cDNA library was titrated on E. coli strain Y1090r on plates containing lmg/ml X-gal, and 0.4 mg/ml IPTG. The cDNA was amplified as plate lysate on E. coli strain Y1090r⁻ at a density of 15,000 plaques per 150mm plate.

### EXAMPLE 3

Screening the lambda-gt 11 cDNA library using specific monoclonal antibody probes.

Ryegrass pollen allergen-specific monoclonal antibodies were developed and characterized by SMART et al. (1983). Sera from patients allergic to ryegrass pollen were kindly provided by Dr David Hill from the Royal Children's Hospital, Melbourne.

The following procedure for screening the lambda-gt 11 expression library is a modification of a previously described method (HUYNH, YOUNG AND DAVIS, 1985). A single colony of E. coli Y1090r- was grown at 37°C with good aeration to OD600 of 0.7 - 0.9, in LB medium containing 100 ug/ml ampicillin and 0.4% (w/v) maltose. The cells were pelletted and resuspended in 10 mM MgSO₄ in 40% of the culture volume. The E. coli Y1090r⁻ cells (0.3 ml) were then infected with approximately 18,000 - 20,000 recombinant phage at 37°C for 15 minutes, plated onto 150mB LB plates in 0.7% (w/v) agarose and incubated at 42°C for 3 hours. The plates were overlayed with dry 132mm nitrocellulose filters presoaked in 10mIPTG then incubated for 6 h at 37°C and the filters removed. A second IPTG-treated filter was placed on the bacterial lawn and the plates incubated overnight at 37°C. Filter plaque lifts were dried at room temperature, and washed with TBS ofr 10 minutes. The TBS was removed and 10ml of TBS containing 10% (w/v) non-fat milk powder was added and the filters were gently agitated for 1 h, drained, rinsed for 30 sec with TBS then washed for 10 minutes with TBS plus 0.1 (v/v) Tween -20 followed by two more washes of TBS for 10 min each. The filters were incubated ro 3 h in TBS containing 2% (w/v) BSA and monoclonal antibodies (ascites) to ryegrass pollen allergens at a dilution of 1:500 with gentle agitation. Following washing in TBS/0.1% (v/v) Tween-20, the filters were incubated ro 1.5 h in TBS containing 2% (w/v) BSA and peroxidase-conjugated affinity - purified anti-mouse IgG at a dilution of 1:500. The filters were washed and developed using fresh chromogenic peroxidase substrate 4-chloro-1-naphthol, 60mg dissolved in 20ml ice-cold methanol, and 80ml TBS containing 0.03% (v/v) H₂O₂. For each filter, 10ml of developing solution was used. After purple spots appeared on the filters, the developing solution was removed and the filters washed with distilled water to stop the reaction.

The developed filter was used to locate specific plaque areas on the plate, corresponding to a positive signal. Positive phage plaques were lifted from the plates as agrose plugs and the eluted phage purified to individual antigen-positive lambda-gt 11 clones by rescreening at lower density on 85mm petri dishes with 82mm nitrocellulose circles. Once plaque purification had been achieved, each of the lambda-gt 11 clones bearing allergen cDNA was plated at low density, and duplicate filter lifts were made. The ability of the recombinant allergen to bind with antisera from allergic patients was detected using the same procedure as described above, except that the overnight filter lift was incubated in the allergic antisera at a dilution of 1:10 whereas the first lift was treated with monoclonal antibodies.

### EXAMPLE 4

### Preparation of recombinant allergenic proteins from lambda-gt 11 recombinant lysogens and Western Blot Analysis.

Bacteria (E. coli strain Y1089) to be lysogenized by the recombinant phage were grown to saturation in LB medium (pH 7.5) containing 0.2% (w.v) maltose at 37°C. One ml of the cells was collected by centrifugation and resuspended in 300 ul of LB medium containing 10mM MgCl₂. The cells (approximately 1 x 10⁸ cells) were infected with about 1 x 10⁹ pfu of lambda-gt 11 recombinant phage containing cDNA inserts coding for the allergenic proteins (e.g. clone 12R and clone 6R) at 32°C for 20 minutes. The infected cells were serially diluted and plated at the density of 100-200 colonies per plate and incubated overnight at 32°C. Individual colonies were spotted onto replicate LB plates, of which one was incubated at 42°C, and the other at 32°C overnight. Recombinant lysogen clones were indicated by growth at 32°C but not at 42°C, and occurred at a frequency of 20% for clone 12R, and 3-4% for clone 6R.

In order to obtain a preparative amount of the recombinant allergenic proteins, a single lysogen colony of Y1089 was inoculated into 10ml of LB medium and incubated 32°C with good aeration until the OD600 reached 0.5. The culture was quickly shifted to a 42°C water bath and incubated for 20 minutes with shaking.

The lac operon repressor was inactivated by addition of 100 ul of 1M IPTG. The culture was then incubated at 37°C for 1 hour allowing the lac Z gene to be expressed and the allergenic proteins to be synthesized as a fusion protein with beta-galactosidase. Cells were harvested by spinning at 3000 rpm for 10 minutes at room temperature, resuspended in 150 ul of Sample buffer and immediately frozen in liquid nitrogen. The cells were lysed by thawing at room temperature. For electrophoretic analysis of proteins, 150 ul of SDS sample buffer containing bromophenol blue tracking dye was added to the freeze-thaw lysate. Samples were boiled for 3 minutes and the insoluble material removed by micro-centrifugation for 3 minutes.

Proteins were resolved by 7-10% (w/v) SDS - polyacrylamide gel electrophoresis and visualised by Coomassie Blue-staining with duplicate samples electroblotted onto nitrocellulose filter using the Bio-Rad Trans Blot apparatus (0.15 amps overnight). Fusion proteins were detected with monoclonal antibodies and visualized using the screening procedure described previously.

### EXAMPLE 5

### Northern analysis.

Total RNA was extracted from pollen, leaf, hydrated seed and root samples as previously described for pollen, and 20 ug RNA/sample electrophoresed in formaldehyde/1.2% (w/v) agarose gels (MANIATIS et al., 1982) run at 70V for 4 hrs in running buffer containing 20mM morpholinopropane sulphonic acid, 5mM sodium acetate and 0.1mm EDTA, to pH 7.0. The RNA's were transferred to nitrocellulose (Hybond C) filters and pre-equilibrated 2 hours at 50°C in hybridization buffer containing 50% (v/v) deionised formamide, 2X SSPE, 7 % (w/v) SDS, 0.5% (w/v) non-fat milk powder, 1% PEG 20,000, and 0.5mg/ml non-homologous herring sperm carrier DNA. Fresh hybridization buffer containing the random primed AP DNA probe was added and incubated at 50°C for overnight hybridization. Filters were washed vigorously in 2X SSC, 0.1% (w/v) SDS for 15 minutes at RT, then 0.5 X SSC, 1% (w/v) SDS at 50°C for 15 minutes, followed by a brief rinse in 0.5x SSC, 0.1% (w/v) SDS, blotted lightly and wrapped in Glad Wrap. Kodak film was exposed for 18 hours at -70°C.

### EXAMPLE 6

### Expression of AP cDNA products reacted with IgE from allergic sera.

The cDNA insert from lambda-gt 11-12R which codes for AP was sub-cloned into the EcoRl site of the plasmid expression vector pGEX where it can be expressed as a fusion protein with glutathione transferase. E. coli infected with this plasmid pGEX-12R or with the non-recombinant vector alone, were grown at a log phase culture, and the bacteria pelleted by centrifugation. These bacteria were lysed and the total proteins separated on SDS-PAGE gel. A western blot shows that only bacteria containing recombinant-plasmids possess a protein component reactive with specific IgE in sera taken from donors known to be allergic to ryegrass pollen. Those results are shown in Figure 6.

### EXAMPLE 7

### Cross-reactivity of AP with homologous allergens from other grass pollen.

AP is a protein of MW 34 kD, and SDS-PAGE shows that other common grasses possess a homologous protein of similar molecular weight. Our results show that these proteins share a common antigenic epitope (detected by monoclonal antibodies), and are allergens in terms of specific IgE- binding. Results are shown in Figure 7. Because of this allergenic similarity, AP is the immunodominant allergen of grass pollen. A consequence is that the cDNA clone 12R can be used as a heterologous probe to isolate the homologous cDNA clones for allergens from other grass pollens.

### REFERENCES:

BALDO, B.A., SUTTON, R., and WRIGLEY, C.W. (1982). Grass allergens with particular reference to cereals. Prog. Allergy 30, 1-66. (Karger, Basel).

BERGER, S.L. and BIRKENMEIER (1979). Inhibition of tractable nucleases with ribonucleoside-vanadyl complexes: isolation of messenger ribonucleic acid from resting lymphocytes. Biochemistry 18; 5143-5149.

BOUSQUET, J. & MICHEL, F.B. (1989) Specific Immunotherapy: A treatment of the Past? Allergy and Clinical Immunology News 1:7-10

BUELL et al. (1985). Nucleic Acids Research 13: 1923-1958.

DOUILLARD, J.Y. and HOFFMAN (1981). Basic facts about hybridomas in Compendium of Immunology, Vol II, ed. by Schwartz.

FORD & BALDO B.A. (1986). A re-examination of Ryegrass (Lolium perenne) pollen allergens. International ARchives of Allergy and Applied Immunology 81; 193-203.

FREIDHOFF, L.R., EHRLICH-KANTZKY, E., GRANT, J.H., MEYERS, D.A., MARSH, D.G. (1986). A study of the human immune response to Lolium perenne (Rye) pollen and its components, Lol pI and Lol pII (Rye I and Rye II). Journal of Allergy and clinical immunology.

GOFF and GOLDBERG (1985). Cell 41: 587-595.

GRAMMER et al., 1984. Persistence of efficacy after a brief course of polymerized ragweed allergens: A controlled study. J. Allergy Clin. Immunol. 73:484-489

GUBLER, V. and HOFFMAN, B.J. (1983). Gene 25, 263.

HILL, D.J., SMART, I.J. and KNOX, R.V. (1979). Childhood asthma and grass pollen aerobiology in Melbourne. Medical Journal of Australia 1, 426-429.

HOWLETT, B.J. and CLARKE, A.E. (1981). Isolation and partial characterization of two antigenic glycoproteins from ryegrass (Lolium perenne) pollen. Biochemical Journal, 197, 695-706.

HOWLETT, B.J. & KNOX, R.B. (1984). Allergic interactions; in Heslop-Harrison J, Linskens, H.F. (eds) Encyclopaedia of Plant Physiology 17, 655-674. Springer, Heidelberg.

HUYNH, T.V., YOUNG, R.A. and DAVIS, R.W. (1985). Constructing and screening cDNA libraries in lambda-gt 10 and lambda-gt 11. In Glover, D.M. (ed); DNA cloning; A practical approach Vol. 1. pp49-78. IRL Press, Oxford.

JOHNSON, P. & MARSH, D.G. (1965. 'Isoallergens' from ryegrass pollen. Nature, 206, 935-

JOHNSON, P. & MARSH, D.G. (1966). Allergens from common ryegrass pollen (Lolium perenne). I. Chemical composition and structure. Immunochemistry 3, 91-100.

KOHLER, G and MILSTEIN, C. (1975). Nature 256: 495-49.

KOHLER, G and MILSTEIN, C. (1976). European JOurnal of Immunology 6: 511-519.

LICHTENSTEIN L.M. et. al. (1983) Int. Cong. of Allergology and Clin. Immunol. J.W. Kerr and .A. Ganderton (eds).

LOWENSTEIN, H. (1978). Quantitive immunoelectrophoretic methods as a tool for the analysis ad isolation of allergens. Prog. Allergy, 25, 1-62. (Karger, Basel).

MANIATIS, T., FRITSCH, E.G. and SAMBROOK, J. (1982). Molecular Cloning; A Laboratory Manual. Cold Spring Harbour Laboratory.

MARSH, D.G., HADDAD Z.H. and CAMPBELL, D.H. (1970). A new method for determining the distribution of allergenic fractions in biological materials: Its application to grass pollen extracts. J. Allergy, 46, 107-121.

MARSH, D.G. (1975). Allergens and the genetics of allergy; in M. Sela (ed), The Antigens, Vol. 3, pp 271-359. (Academic Press Inc., London, New York).

MARSH, D.G., MILNER, F.M. and JOHNSON, P. (1966). The allergenic activity and stability of purified allergens from the pollen of common ryegrass. International ARchives of Allerby 29, 52-

MESSING, J. (1983). New M13 vectors for cloning. Methods in Enzymology - Recombinant DNA techniques 101, Part C. pp. 20-78.

MESSING, J. & VIEIRA, J. (1982), A new pair of M13 vectors for selecting either DNA strand of double-digest restriction fragments. Gene; 19, 269-276.

MILLER, J.H. (1972). Experiments in Molecular Genetics, Cold Spring Harbor Laboratory, Cold Spring harbor, New York.

READING, J. (1982), Journal of Immunological Methods 53: 261-291

SINGH, M.B. & KNOX, R.B. (1985). Grass pollen allergens: Antigenic relationships detected using monoclonal antibodies and dot blotting immunoassay. International Archives of Allergy and Applied Immunology 78, 300-304.

SMART, I.J., HEDDLE, R.J. ZOLA, M., BRADLEY, J. (1983). Development of monoclonal antibodies specific for allergenic components in ryegrass (Lolium perenne) pollen. International ARchives of Allergy and Applied Immunology 72 243-248.

SMART, I.J. & KNOX, R.B. (1979). Rapid batch fractionation of ryegrass pollen allergens. International Archives of Allergy and Applied Immunology 62, 173-187.

WATSON, J. (1976). Molecular Biology of the Gene, 3rd Ed. Benjamin-Cummings, Menlo Park, California.

## Claims

1. A DNA sequence exhibiting at least 60% homology with the sequence depicted in Fig 5, and encoding an allergenic protein from pollen of the family Poaceae.

2. A DNA sequence as claimed in claim 1, wherein the pollen is pollen of Lolium perenne.

3. A DNA sequence exhibiting at least 60% homology with the sequence depicted in Fig 5, and encoding an allergenic protein from a grass pollen which is allergenically cross reactive with an allergenic pollen protein from Lolium perenne as shown by IgE binding studies.

4. An expression vector comprising a DNA sequence as defined in any one of claims 1 to 3.

5. A host cell transformed to express a protein or polypeptide encoded by a DNA sequence as defined in any one of claims 1 to 3.

6. A host cell as claimed in claim 5 which is E coli.

7. A cDNA comprising a DNA sequence as defined in any one of claims 1 to 3 and obtainable by:
extracting RNA from ryegrass pollen, and selecting Poly (A+) mRNA by affinity chromatography to serve as a template for synthesis of single-stranded cDNA;
synthesizing and isolating double stranded cDNA;
constructing a lambda-gt 11 cDNA expression library;
screening the lambda-gt 11 cDNA expression library after induction with IPTG using monoclonal antibodies characterized by *Smart et al., Int Archives of Allergy and Appl Immunol., 72, 243-248* to probe duplicate filter lifts and identifying and isolating positive phage plaques;
plating the plaques at low density and conducting duplicate filter lifts of said plaques;
screening said phage plaques containing clones producing protein allergen by incubating said filter lifts with allergic antisera or with said Smart et al monoclonal antibodies and identifying those clones which are positive when screened with monoclonal antibodies and positive when screened with allergic antisera;
recovering the cDNA insert from the allergic antisera and antibody positive phage and identifying by size analysis inserts of approximately 1200 base pairs;
inserting the said inserts into a plasmid vector for sequencing and expression;
sequencing said cDNA; and
comparing the restriction map of the sequenced DNA to the cDNA restriction map shown in Fig 9, and identifying those clones having substantially the same restriction map as that shown in Fig 9.

8. A method for isolating and identifying DNA encoding an allergenic protein of grass pollen comprising the step of using a cDNA of claim 7 as a heterologous probe to isolate the homologous cDNA clones of protein allergens from grass pollen.

9. The use of a DNA sequence as claimed in any one of claims 1 to 3 in the production of a protein or polypeptide encoded by the said DNA sequence.

10. The use of a DNA sequence as claimed in any one of claims 1 to 3 or a cDNA as claimed in claim 7 as a probe for isolating and identifying nucleic acid encoding the said allergenic protein or polypeptide.

## Patentansprüche

1. Eine DNA-Sequenz, die mindestens 60% Homologie mit der Sequenz aus Figur 5 zeigt und ein allergenes Protein aus Pollen der Familie *Poaceae* kodiert.

2. Eine DNA-Sequenz gemäß Anspruch 1, wobei der Pollen Pollen von *Lolium perenne* ist.

3. Eine DNA-Sequenz, die mindestens 60% Homologie mit der Sequenz in Figur 5 aufweist und ein allergenes Protein von einem Graspollen kodiert, das allergen-kreuzreaktiv ist mit einem allergenen Pollenprotein von *Lolium perenne* wie durch IgE-Bindungsstudien gezeigt ist.

4. Ein Expressionsvektor, umfassend eine DNA-Sequenz gemäß einem der Ansprüche 1 bis 3.

5. Eine Wirtszelle, transformiert, um ein Protein oder Polypeptid kodiert durch eine DNA-Sequenz, gemäß einem der Ansprüche 1 bis 3, zu exprimieren.

6. Eine Wirtszelle gemäß Anspruch 5, die *E. coli* ist.

7. Eine cDNA umfassend eine DNA-Sequenz gemäß einem der Ansprüche 1 bis 3 und erhältlich durch:
- Extraktion von RNA aus Raigraspollen und Selektion von Poly-(A+)-mRNA durch Affinitätschromatographie, um als Templat zur Synthese einer einzelsträngigen cDNA zu dienen;
- Synthese und Isolieren von doppelsträngiger cDNA;
- Konstruktion einer Lambda-gt-11 cDNA-Expressionsgenbank;
- Screening der Lambda-gt-11 cDNA-Expressionsgenbank nach Induktion mit IPTG unter Verwendung von monoklonalen Antikörpern, charakterisiert durch *Smart et al., Int. Archives of Allergy and Appl. Immunol.,* 72, 243-248, um duplizierte Filterabzüge zu sondieren und Identifizieren und Isolieren positiver Phagenplaques;
- Platieren der Plaques mit einer niedrigen Dichte und Aufbringen der duplizierten Filterabzüge auf diese Plaques;
- Screening dieser Phagenplaques, die Klone enthalten, die ein Protein-Allergen produzieren durch Inkubieren dieser Filterabzüge mit aller genen Antisera oder mit monoklonalen Antikörpern nach Smart et al. und Identifizieren solcher Klone, die positiv sind, wenn sie mit monoklonalen Antikörpern gescreent werden und positiv sind, wenn sie mit allergenen Antisera gescreent werden;
- Gewinnung des cDNA-Inserts aus den allergenen Antisera und Antikörper-positiven Phagen und Identifizierung durch Größenanalyse der Inserts von ungefähr 1200 Basenpaaren;
- Inserierung dieser Inserts in einen Plasmidvektor zur Sequenzierung und Expression;
- Sequenzierung dieser cDNA; und
- Vergleich der Restriktionskarte der sequenzierten DNA mit der cDNA-Restriktionskarte gezeigt in Figur 9 und Identifizierung solcher Klone, die im wesentlichen die gleiche Restriktionskarte haben, die in Figur 9 gezeigt ist.

8. Ein Verfahren zur Isolierung und Identifizierung von DNA, die ein allergenes Protein von Graspollen kodiert, umfassend, den Schritt der Verwendung einer cDNA des Anspruchs 7 als ein heterologes Sondenmolekül, um die homologen cDNA-Klone von Protein-Allergenen aus Graspollen zu isolieren.

9. Die Verwendung einer DNA-Sequenz gemäß einem der Ansprüche 1 bis 3 für die Herstellung eines Proteins oder Polypeptids, kodiert durch diese DNA-Sequenz.

10. Die Verwendung einer DNA-Sequenz gemäß einem der Ansprüche 1 bis 3 oder einer cDNA gemäß Anspruch 7 als ein Sondenmolekül zur Isolierung und Identifizierung von Nucleinsäuren, die dieses allergene Protein oder Polypeptid kodieren.

## Revendications

1. Séquence d'ADN présentant au moins 60 % d'homologie avec la séquence représentée figure 5, et codant pour une protéine allergène du pollen de la famille des Poaceae.

2. Séquence d'ADN selon la revendication 1, dans laquelle le pollen est du pollen de Lolium perenne.

3. Séquence d'ADN présentant au moins 60 % d'homologie avec la séquence représentée figure 5, et codant pour une protéine allergène de pollen de graminées qui réagit allergéniquement de manière croisée avec une protéine de pollen allergène de Lolium perenne comme montré dans les études de liaison des IgE.

4. Vecteur d'expression comprenant une séquence d'ADN telle que défini dans l'une quelconque des revendications 1 à 3.

5. Cellule hôte transformée pour exprimer une protéine ou un polypeptide codé par une séquence d'ADN telle que définie dans l'une des revendications 1 à 3.

6. Cellule hôte selon la revendication 5 qui est E. Coli.

7. ADNc comprenant une séquence d'ADN telle que définie dans l'une quelconque des revendications 1 à 3 et pouvant être obtenue par :
- extraction de l'ARN à partir du pollen de ray-grass, et sélection des ARNm poly(A+) par chromatographie d'affinité pour servir de matrice pour la synthèse d'ADNc simple-brin ;
- synthèse et isolement d'ADNc double-brin,
- construction d'une banque d'expression d'ADNc dans lambda-gt 11 ;
- criblage de la banque d'expression d'ADNc dans lambda-gt 11 après induction avec IPTG en utilisant des anticorps monoclonaux caractérisés par *Smart et al., Int Archives of Allergy and Appl Immunol., 72, 243-248* pour sonder des transferts de filtres dupliqués et identification et isolement des plages de phages positives ;
- étalement des plages à basse densité et transfert desdites plages sur filtres dupliqués ;
- criblage desdites plages de phages contenant des clones produisant la protéine allergène par incubation desdits transferts de filtres avec des anti-sérums allergiques ou avec lesdits anticorps monoclonaux de Smart et al et l'identification des clones qui sont positifs lors du criblage avec des anticorps monoclonaux et positifs lors du criblage avec les anti-sérums allergiques ;
- récupération de l'insert d'ADNc à partir des phages positifs avec les anti-sérums et les anticorps allergiques et identification par analyse de taille des inserts d'approximativement 1200 paires de bases ;
- insertion desdits inserts dans un vecteur plasmidique pour le séquençage et l'expression ;
- séquençage dudit ADNc ; et
- comparaison de la carte de restriction de l'ADN séquencé avec la carte de restriction de l'ADNc telle que représentée figure 9, et
- identification des clones ayant essentiellement la même carte de restriction que celle représentée figure 9.

8. Procédé pour isoler et identifier l'ADN codant pour une protéine allergène de pollen de graminées comprenant l'étape consistant à utiliser un ADNc selon la revendication 7 comme sonde hétérologue pour isoler les clones d'ADNc homologue des allergènes protéiques à partir de pollen de graminées.

9. Utilisation d'une séquence d'ADN selon l'une des revendications 1 à 3 pour la production d'une protéine ou d'un polypeptide codé par ladite séquence d'ADN.

10. Utilisation d'une séquence d'ADN selon l'une quelconque des revendications 1 à 3 ou d'un ADNc selon la revendication 7 comme sonde pour isoler et identifier un acide nucléique codant pour ladite protéine ou ledit polypeptide allergène.
